# EUROPEAN PATENT APPLICATION

(11) **EP 2 365 329 A1**
(43) Date of publication of application: **14.09.2011**
(21) Application number: 10002653.3
(22) Date of filing: 12.03.2010
(51) Int. Cl.: G01N 33/487, C12Q 1/00

(54) **Method of manufacturing planar bio-test strip and product thereof**

(71) Applicant: Hivox Biotek Inc., Taipei County 24158 (TW)
(72) Inventor: Tuan, Wei, 11666 Taipei City (TW)
(74) Representative: Zeitler - Volpert - Kandlbinder

(57) **Abstract**

A planar bio-test strip 10A, 10C includes a carrier plate 11A, 11C, a conductive circuit 13A, 13C formed on the carrier plate 11A, 11C by evaporation, a testing electrode terminal 131 A, 131C and an electrode circuit terminal 132A, 132C formed at front and rear ends of the conductive circuit 13A, 13C respectively, and an enzyme reacting area 16A, 16C coupled to a front end of the testing electrode terminal 131A, 131C. A manufacturing method of the planar bio-test strip 10A, 10C includes (1) using a plastic film 31 as a carrier plate 11A, 11C, and constructing a shadow mask 12A, 12C by printing; (2) evaporating the carrier plate 11 A, 11C printed with the shadow mask 12A, 12C to form a conductive electroplated layer 17A, 17C; (3) removing a block of the conductive electroplated layer 17A, 17C covered by the shadow mask 12A, 12C by a physical method to expose the conductive circuit 13A, 13C; (4) setting an enzyme in an enzyme reacting area 16A, 16C; (5) attaching a cover film 15A, 15C; such that the test strip 10A, 10C can be mass produced with low costs, and enhanced quality, stability and market competiveness.

## Description

### BACKGROUND OF THE INVENTION

### 1. Field of the Invention

The present invention relates to a method of manufacturing a planar bio-test strip and a product thereof, and more particularly to a test strip used for testing and measuring physiological conditions of biological liquids such as testing and measuring blood glucose and cholesterol in human blood or urine, and the test strip product provides higher stability and precision, and the manufacturing process of the product incurs lower costs.

### 2. Description of the Related Art

As our prolonged life span is closely related to the advancement of medical technologies, a number of latent symptoms can be examined and confirmed at an early stage by medical instruments to achieve early-stage prevention and treatment effects. A blood glucose test described below is used for example, wherein a small blood sample is collected from a human body first, and then the blood is dropped in a blood glucose enzyme area of a test strip for performing a reaction and the blood is analyzed by a blood glucose instrument to examine and measure a blood glucose level, so that users can discover any abnormal blood glucose level and take necessary treatments at an early stage in order to prevent critical illness.

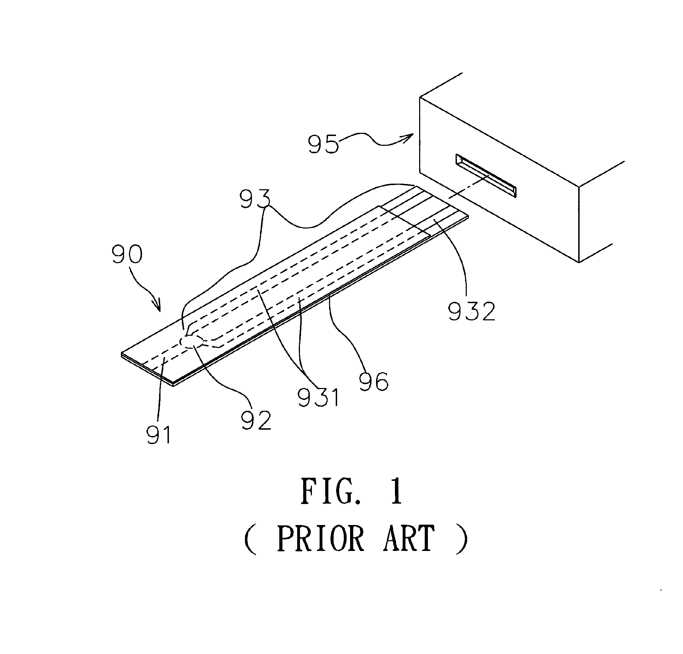

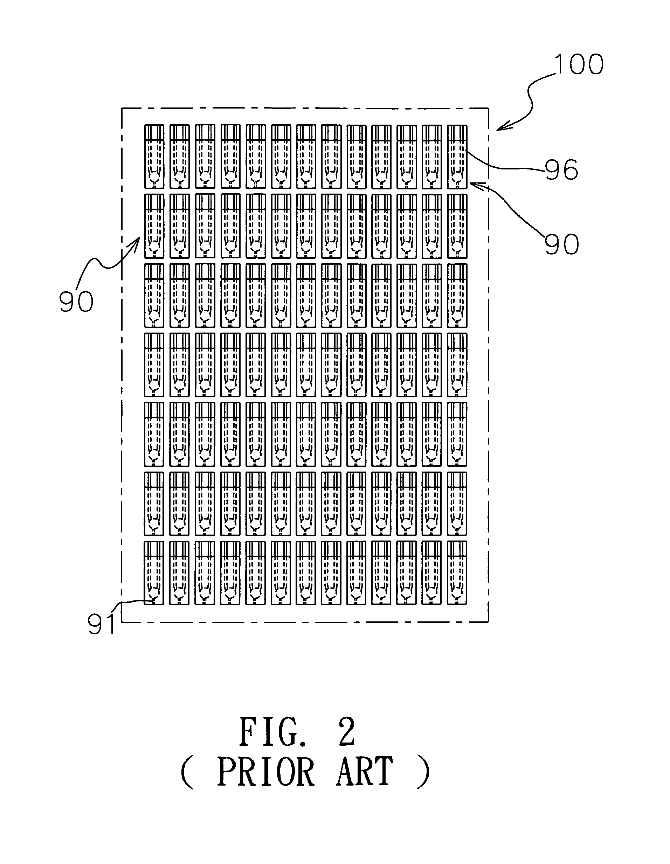

With reference to FIGS. 1 and 2 for a conventional blood glucose test strip, the test strip 90 adopts a single plastic plate (made of polyimide) as a substrate 100, and then an electrically conductive graphite material is used for a silk-screen carbon ink printing process or a copper clad is formed on a surface of the substrate 100 by photolithography and an etching process to produce a conductive circuit 93, wherein the conductive circuit 93 includes a testing electrode terminal 931 at its front section and an electrode circuit terminal 932 at its rear section, and an enzyme reacting area 92 (containing an enzyme) and a siphon passage 91 are connected sequentially to the front end of the testing electrode terminal 931, and then a cover film 96 is coated, and the electrode circuit terminal 932 is exposed, and finally the test strips are cut into an appropriate size.

When use, a blood sample in contact with the siphon passage 91 is collected and sucked to the enzyme reacting area 92 by a capillary action, so that glucose and enzyme in the blood are gone through an reaction to produce an electron stream, and the electron stream is passed through the testing electrode terminal 931 to the electrode circuit terminal 932, and the electrode circuit terminal 932 is connected to a blood glucose meter 95, and the blood glucose meter 95 is provided for measuring a current passing through the electrode circuit terminal 932, converting the current into an electric signal, and finally converting the electric signal into a corresponding blood glucose concentration value.

Although the foregoing conventional blood glucose test strip 90 can achieve the effect of measuring a blood glucose level, its manufacturing method still has the following shortcomings.
1. The single soft plastic plate 100 is used as the substrate, and the testing electrode terminal 931 at the front section and the electrode circuit terminal 932 at the rear section are formed on the substrate by the silk-screen carbon ink printing method. Since the testing electrode terminal 931 and the electrode circuit terminal 932 have a longer length and a larger surface area, therefore the manufacture is relatively unstable and a non-uniform electric resistance may result easily.
2. To overcome the aforementioned unstable manufacturing condition, most manufacturers print a conductive silver ink at the carbon ink substrate. Although the stability can be improved by the silver ink, the silver ink incurs a higher material cost and the printing process requires a higher precision of thickness and a more complicated drying process.

Obviously, the aforementioned conditions are unfavorable to the manufacture of the test strips.

Therefore, a conductive circuit of a blood glucose test strip manufactured by photolithography is disclosed in U.S. Pat. No. 7,465,597, wherein a photo mask is manufactured precisely from a designed conductive circuit pattern and duplicated onto a substrate, and the principle of optical imaging is applied to project the pattern onto the substrate. Since a light emitted from a light source and passing through a transparent area of the photo mask can travel continuously, an image is formed on a surface of the substrate, wherein the surface of the substrate must be cleaned and processed in advance, and a photoresist is coated onto the surface of the substrate, such that the light passing through the photo mask can be reacted with the photoresist, and this process is generally called "exposure". After the exposure takes place, the substrate goes through a development process, and then a method of evaporating metal is applied for depositing a gaseous metal onto the conductive circuit without being coated by the photoresist and onto a mask layer of the photoresist, and then a chemical method (such as dissolving and etching by acid) is applied to remove the photoresist, so that the metal coated onto the photoresist is removed, and a metal circuit is remained to form the desired conductive circuit, and then the steps of setting an enzyme reacting area, covering the test strip, and cutting the test strip into an appropriate size are carried out.

Although photolithography used in the aforementioned manufacturing method of a conductive circuit of a blood glucose test strip does not have the problems of a low manufacturing precision or a high level of difficulty of controlling the impedance due to the large surface area of the carbon ink material, yet it requires a highly accurate and precision micro-electromechanical system (MEMS) and circuit board layout, so that the production cost is still relatively high. On the other hand, chemicals are used for removing the photoresist in an acid etching process, and thus the acidic remnant will destroy the enzyme activation. With a poor process control, problems including enzyme degeneration and environmental pollution may occur. Obviously, such manufacturing method is not good enough, and it is a major issue for related manufacturers to improve the manufacturing method of the conventional blood glucose test strip.

In view of the shortcomings of the conventional planar bio-test strip, production, and the conventional manufacturing method, the inventor of the present invention based on years of experience in the related industry to conduct extensive researches and experiments, and finally invented a method of manufacturing a planar bio-test strip with the advantages of high precision and stability as well as low cost.

### SUMMARY OF THE INVENTION

Therefore, it is a primary objective of the present invention to provide a method of manufacturing a planar bio-test strip and a product thereof, wherein a plastic film is used as a carrier plate (and the carrier plate is made of a rigid material and printed onto an object), so that the invention can achieve the effects of providing a quick production, lowering the cost, simplifying the manufacturing procedure and enhancing the quality, stability and market competiveness of the product.

Another objective of the present invention is to provide a method of manufacturing a planar bio-test strip and a product capable of meeting environmental protection requirements, wherein a printing method is used for processing a shadow mask (made of a non-toxic water-based oil ink), and an evaporation process is applied to coat a conductive electroplated layer, and then a physical method (such as a water rinsing process) is used to remove the conductive electroplated layer coated with a shadow mask to develop a conductive circuit that is not covered by the shadow mask. The whole manufacturing procedure just involves physical processes only, and thus the environmental protection requirements can be satisfied, and the impedance and electrical stability can be controlled easily, and the precision and sensitivity of detecting micro currents in an electro biochemical reaction can be improved.

To achieve the foregoing objectives, the present invention provides a manufacturing method of a planar bio-test strip and a product thereof, wherein a carrier plate is made of a plastic film, and a conductive circuit is formed on the carrier plate by an evaporation process, and a testing electrode terminal and an electrode circuit terminal are formed at front and rear sections of the conductive circuit respectively and electrically conducted with each other, and an enzyme reacting area is coupled to a front end of the testing electrode terminal, and a cover film is covered onto the carrier plate, wherein the cover film is covered onto the carrier plate, but the electrode circuit terminal is exposed to facilitate a test.

To achieve the foregoing objective, the present invention provides a manufacturing method of a planar bio-test strip, wherein the method comprises the steps of: forming a conductive circuit of a testing electrode terminal on the carrier plate by coating or printing an electrically conductive material (such as a carbon ink and a conductive silver ink) that cannot be chemically reacted with an enzyme; coating an evaporated metal conductor onto the electrode circuit terminal and onto the testing electrode terminal away from the enzyme reacting area, such that the electrode circuit terminal and the testing electrode terminal are electrically conducted, and upper and lower coupling electrodes are formed at the testing electrode terminal away from the enzyme reacting area. Since the testing electrode terminal in contact with the enzyme reacting area is made of an electrically conductive material that is not chemically reacted with the enzyme easily, therefore a test can have a higher stability, and the test result will not be affected by environmental factors or storage time, and the control of the coefficient of variation (CV) will be easier.

To achieve the foregoing objective, the present invention adopts a sheet plastic film for the carrier plate and prints a massive quantity of unit products.

To achieve the foregoing objective, the present invention adopts a whole roll of cylindrical plastic film for the carrier plate, and prints continuous unit products by roll to roll.

To achieve the foregoing objective, the present invention provides a manufacturing method of a planar bio-test strip, and the method comprises: (1) using at least a single layer of plastic film as a carrier plate, and constructing a shadow mask by a printing method, wherein the shadow mask is provided for developing patterns of a testing electrode terminal and an electrode circuit terminal; (2) forming a conductive circuit by an evaporation process, wherein a continuous conductive electroplated layer is covered completely onto the side printed with the shadow mask by an evaporation process; (3) removing the conductive electroplated layer covered by the shadow mask, wherein the conductive electroplated layer covered by the shadow mask is removed by a physical method, and the whole conductive circuit is exposed, and the conductive circuit includes the testing electrode terminal and the electrode circuit terminal; (4) setting an enzyme in an enzyme reacting area of the carrier plate; and (5) attaching a cover film onto the carrier plate, and exposing the electrode circuit terminal.

To achieve the foregoing objective, the present invention provides a manufacturing method of a planar bio-test strip, and the method comprises: (1) using at least a single layer of plastic film as a carrier plate, and then coating or printing an electrically conductive material (such as a carbon ink and a conductive silver ink) that does not react with an enzyme easily to form a testing electrode terminal; (2) constructing a shadow mask by a printing method, wherein the shadow mask is provided for developing a pattern of the electrode circuit terminal; (3) forming a conductive circuit by an evaporation process; after the evaporated metal conductor on the carrier plate of the shadow mask is completed and covered onto the testing electrode terminal and the electrode circuit terminal away from the enzyme reacting area, such that the carrier plate is covered with a continuous conductive electroplated layer, and the electrode circuit terminal and the testing electrode terminal are electrically conducted, and upper and lower coupling electrodes are formed on the testing electrode terminal; (4) removing a block of the conductive electroplated layer covered by the shadow mask, wherein the block of the conductive electroplated layer covered by the shadow mask is removed by a physical method, and the conductive circuit is exposed completely, and the conductive circuit includes a testing electrode terminal and an electrode circuit terminal; (5) setting an enzyme in enzyme reacting area of the carrier plate; and (6) attaching a cover film onto the carrier plate, and exposing the electrode circuit terminal.

To achieve the foregoing objective, the present invention provides a manufacturing method, and the method further comprises: forming the carrier plate by a whole roll of cylindrical plastic film, and continuously printing unit products by roll to roll to achieve the effects of providing a quick mass production, lowering the cost, simplifying the manufacturing procedure, and enhancing the quality, stability and market competiveness of the product.

The technical characteristics of the present invention will become apparent with the detailed description of the preferred embodiments and the illustration of the related drawings.

### BRIEF DESCRIPTION OF THE DRAWINGS

FIG. 1 is a cross-sectional view of a structure of a conventional blood glucose test strip;
FIG. 2 is a schematic view of a manufacturing process of a conventional blood glucose test strip;
FIGS. 3A to 3D are schematic views of a planar bio-test strip in accordance with a first preferred embodiment of the present invention;
FIG. 4 is a flow chart of a manufacturing method of a planar bio-test strip in accordance with a first preferred embodiment of the present invention;
FIG. 5 is a schematic view of manufacturing a planar bio-test strip in accordance with a first preferred embodiment of the present invention;
FIG. 6 is a schematic view of arranging a planar bio-test strip in accordance with a first preferred embodiment of the present invention;
FIGS. 7A to 7E are schematic views of a planar bio-test strip in accordance with a second preferred embodiment of the present invention; and
FIG. 8 is a flow chart of a manufacturing method of a planar bio-test strip in accordance with a second preferred embodiment of the present invention.

### DETAILED DESCRIPTION OF THE PREFERRED EMBODIMENTS

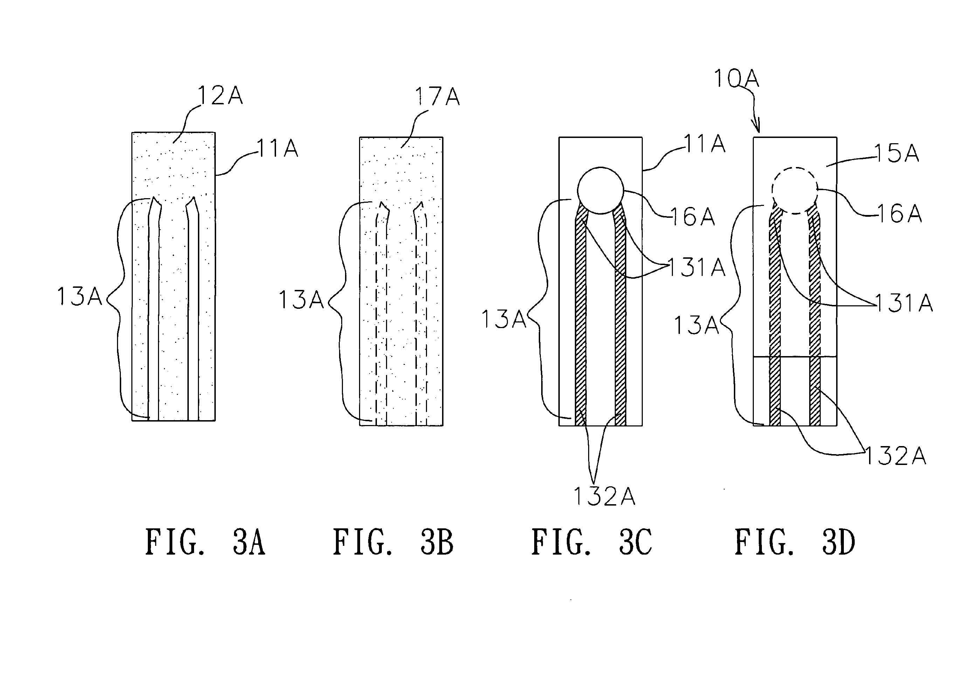

With reference to FIGS. 3C and 3D for a planar bio-test strip in accordance with a first preferred embodiment of the present invention, the bio-test strip 10A comprises a carrier plate 11A, wherein the carrier plate 11A is made of a single layer, a plurality of layers of a sheet, or a whole roll of cylindrical continuous plastic film (such as PET film, PC film, PTFE film, Nylon film, polycarbonate film, polystyrene film, styrene film, acrylonitrile film or acrylonitrile butadiene styrene (ABS) film or any other rigid materials (wherein the whole roll cylindrical plastic film is used in this preferred embodiment), but persons ordinarily skilled in the art can use other equivalent materials instead, and the carrier plate 11 A includes a conductive circuit 13A formed by an evaporation process, and a testing electrode terminal 131A and an electrode circuit terminal 132A are formed at front and rear ends of the conductive circuit 13A respectively, and the front end of the testing electrode terminal 131A is coupled to an enzyme reacting area 16A, and an enzyme is set in the enzyme reacting area 16A, and the carrier plate 11 A includes a cover film 15A, wherein the electrode circuit terminal 132A is exposed to facilitate performing a test.

With reference to FIGS. 3A and 3B for a manufacture of the planar bio-test strip 10A, a shadow mask 12A is printed onto the carrier plate 11A, and patterns of a conductive circuit 13A are exposed by the shadow mask 12A (wherein an intaglio method is used in this preferred embodiment; in other words, patterns of the testing electrode terminal and the electrode circuit terminal are coated or printed); applying an evaporation process to form a conductive electroplated layer 17A, and removing a block of the conductive electroplated layer 17A covered by the shadow mask 12A by a physical method (such as a water rinsing method), and exposing the testing electrode terminal 131A and the electrode circuit terminal 132A, wherein the shadow mask 12A of the preferred embodiment is made of a non-toxic water-based oil ink by a printing method.

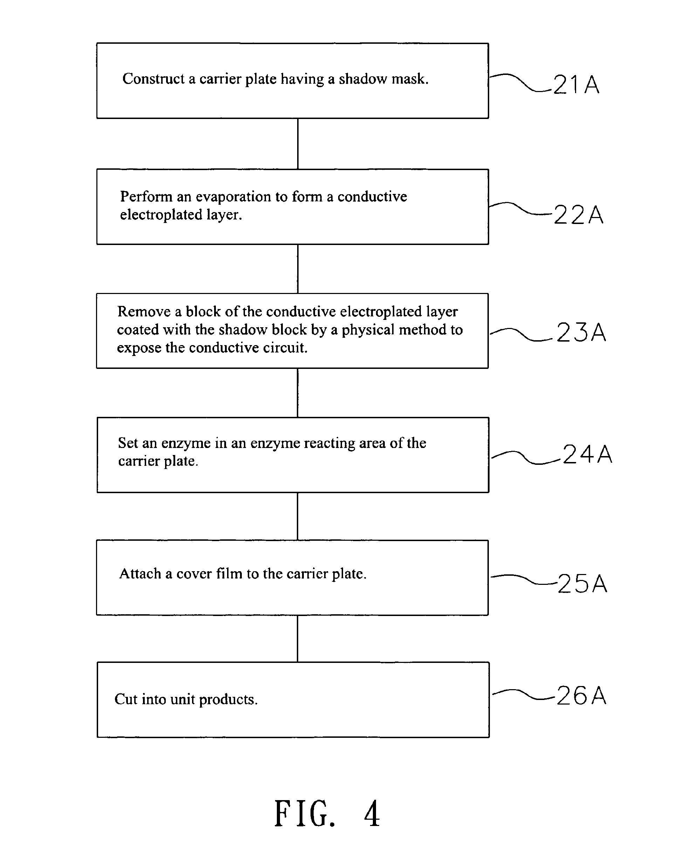

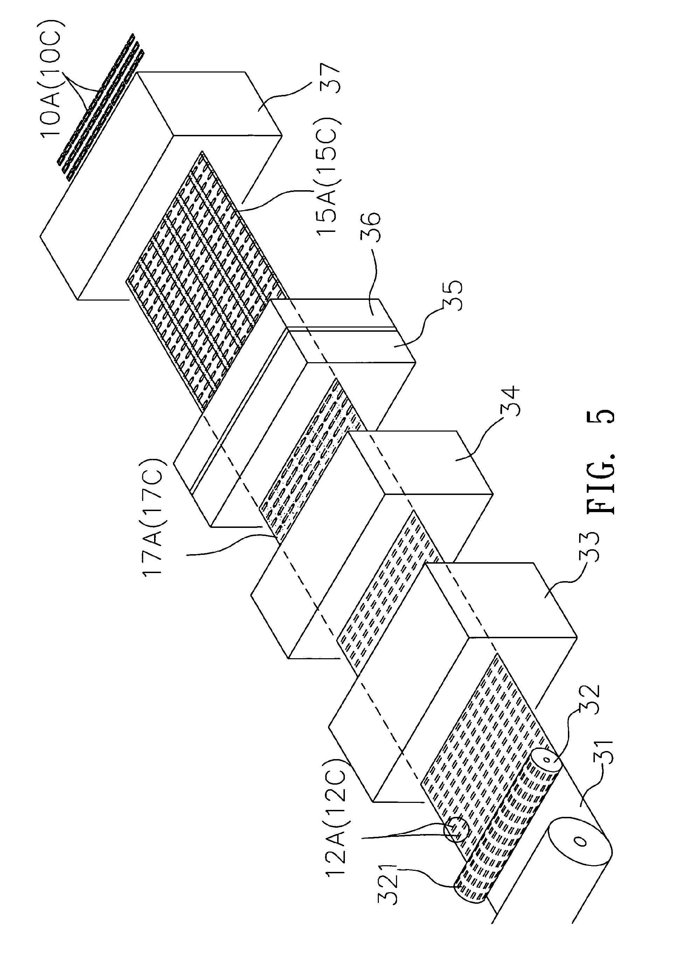

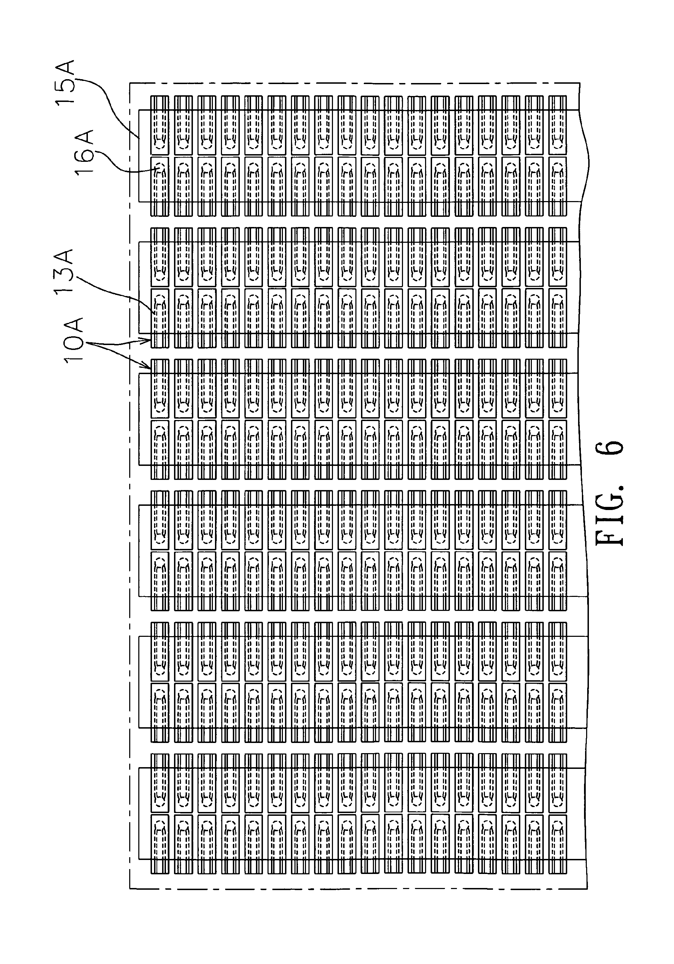

With reference to FIGS. 4 to 6 for a manufacturing method of the aforementioned planar bio-test strip 10 in accordance with the present invention, the manufacturing method comprises:
(1) constructing a shadow mask 12A by using a sheet of plastic film or a whole roll of cylindrical plastic film (which is roll to roll in this preferred embodiment by means of a continuous mass printing method (21A) (wherein the printing method is a silk screen printing, or a gravure roller printing in accordance with this preferred embodiment, and a whole roll of cylindrical plastic film 31 (made of a rigid material such as a PET film, PC film, PTFE film, nylon film, polycarbonate film, polystyrene film, styrene film, acrylonitrile film or acrylonitrile butadiene styrene (ABS) film) is used as the carrier plate 11A (as shown in FIG. 3A), and the carrier plate 11A is printed continuously by a gravure roller printing process that uses a gravure roller 32 (or a silk screen printing, but persons ordinarily skilled in the art can use other equivalent printing methods instead), wherein the gravure roller 32 includes a plurality of pattern meshes 321 for accommodating a non-toxic water-based oil ink, and the carrier plate 11A is printed with a shadow mask 12A;
(2) evaporating to form a conductive electroplated layer (22A), wherein after the carrier plate 11A printed with the shadow mask 12A is baked and dried by a bake-dry equipment 33, the carrier plate 11A is put into a vacuum evaporator 34 for performing an evaporation process, and the evaporation process passes the carrier plate 11A through an evaporation roll coater and winds the carrier plate 11A onto a film coiling station (not shown in the figure), and then a vacuum pump is used for performing a vacuum process, and an evaporation source is heated, such that a highly pure conductive material (such as gold, silver, copper and aluminum) is melted and evaporated into a gas state at a high temperature, and then a film winding system is turned on, such that if the film is moving at a predetermined speed, a stop plate will be opened to deposit gaseous conductive micro-particles onto a surface of the moving carrier plate 11A, and coat a continuous conductive electroplated layer 17A onto the carrier plate 11A, after a cooling process takes place; in the evaporation process, the speed of evaporating the electrically conductive material (such as gold, silver, copper and aluminum) can be controlled, such that the moving speed of the carrier plate 11 A and the vacuum level inside the evaporation chamber can be used for controlling the thickness of the conductive electroplated layer 17A, and the manufacturing quality of the testing electrode terminal 131A and the electrode circuit terminal 132A can be controlled precisely;
(3) removing the conductive electroplated layer 17A covered by the shadow mask 12A (23A), wherein the carrier plate 11A used in the evaporation process is processed by a washing equipment 35 (such as a water rinsing machine) to wash away the block of the conductive electroplated layer 17A covered by the shadow mask 12A, and the exposed portion includes the testing electrode terminal 131A and electrode circuit terminal 132A;
(4) setting an enzyme into an enzyme reacting area (24A) of the carrier plate, wherein the carrier plate 11A is passed through an enzyme printing equipment 36 for setting the enzyme on the carrier plate 11A (or the enzyme reacting area 16A), and the enzyme can be set by a gravure, silk screen or jet printing or a titration, and the thickness of the enzyme can be set according to the depth of the gravure if the gravure printing is adopted in the invention, so that a better reaction result of the enzyme can be achieved to obtain a more accurate test;
(5) attaching a cover film 15A onto the carrier plate 11A (25A), wherein a continuous printing adhesive material (such as a liquid adhesive and an acrylic adhesive) is placed on the carrier plate 11A and attached to the cover film 15A correspondingly, and the electrode circuit terminal 132A is exposed to facilitate performing a test, and it is noteworthy to point out that the enzyme reacting areas 16A (or the testing electrode terminals 131A) of the plastic film 31 are disposed adjacent and opposite to one another as shown in FIG. 6, such that two rows of cover films 15A can be attached simultaneously to improve the manufacturing efficiency; and
(6) cutting the plastic film 31 into unit products (26A), wherein the plastic film 31 installed with related components is entered into a cutting machine 37 for a cutting procedure to produce a plurality of planar bio-test strip 10A (blood glucose detector) products.

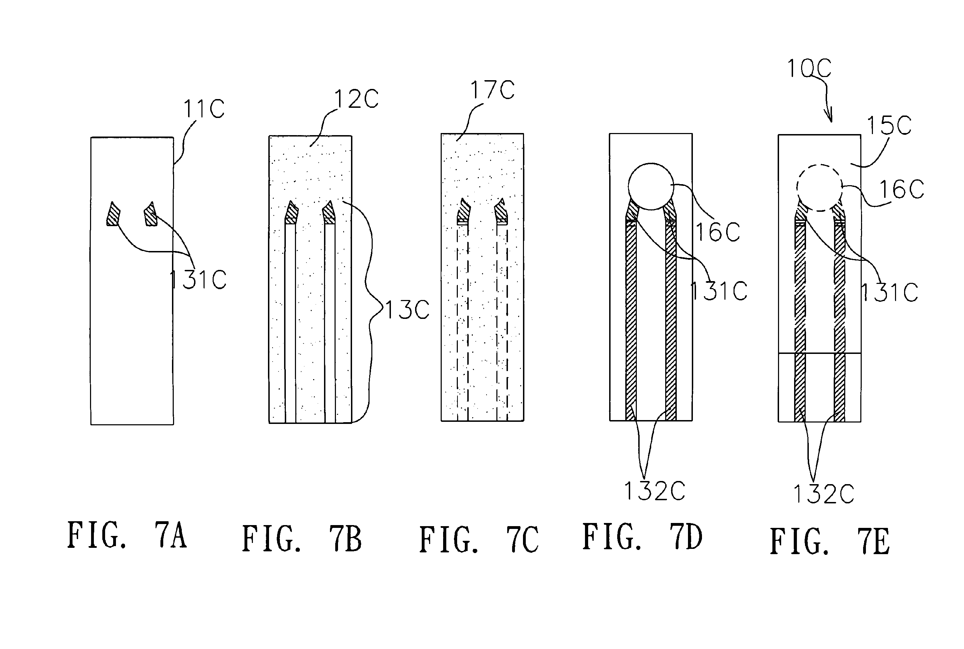

With reference to FIGS. 7A to 7E for a planar bio-test strip in accordance with a second preferred embodiment of the present invention, the bio-test strip 10C comprises a carrier plate 11C (with the same structure of the first preferred embodiment), and a conductive circuit 13C installed on the carrier plate 11C and having a testing electrode terminal 131C and an electrode circuit terminal 132C, wherein the testing electrode terminal 131C is formed by coating or printing an electrically conductive material (such as a carbon ink and a conductive silver ink) that does not chemically react with the enzyme easily, and then covering an evaporated metal conductor (made of a material such as gold, silver, copper and aluminum) onto the electrode circuit terminal 132C and the testing electrode terminal 131C away from the enzyme reacting area 16C, such that the electrode circuit terminal 132C and the testing electrode terminal 131C are electrically conducted, and upper and lower coupling electrodes are formed on the testing electrode terminal 131C, and the testing electrode terminal 131C in contact with the enzyme reacting area 16C is made of a stable electrically conductive material, and thus the testing electrode terminal 131C will not react chemically with the enzyme in the enzyme reacting area 16C easily, and the test conducted by the bio-test strip of the invention provides a higher stability, and the test result will not be changed easily by environmental factors or storage time, and the coefficient of variation (CV) can be controlled easily.

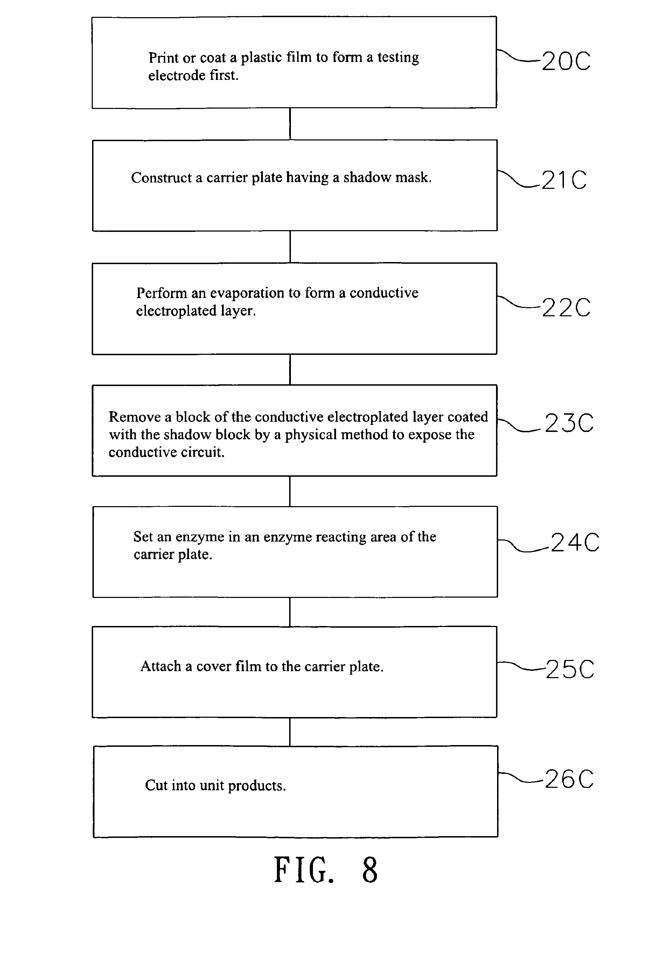

With reference to FIG. 8 for a manufacturing method in accordance with a second preferred embodiment of the present invention, the manufacturing method comprises:
(1) printing or coating a plastic film to form a testing electrode in advance (20C), wherein a sheet of plastic film or a whole roll of cylindrical plastic film is used as the material for making the carrier plate, and printing or coating an electrically conductive material that does not reacted chemically with an enzyme easily onto the plastic film to form a testing electrode terminal;
(2) constructing a carrier plate having a shadow mask by a printing method (21C) wherein a shadow mask 12C is printed onto the carrier plate 11C, and the shadow mask is coupled to the testing electrode terminal 131C;
(3) performing an evaporating process to form a conductive electroplated layer (22C), wherein the carrier plate 11C printed with the shadow mask 12C is processed by a bake-dry procedure and then by an evaporation process, such that the carrier plate 11C with the shadow mask 12C are covered onto the electrode circuit terminal 12C and the testing electrode terminal 131C is situated at a position away from the enzyme reacting area 16C, such that the electrode circuit terminal 132C and the testing electrode terminal 131C are electrically conducted, and upper and lower coupling electrodes are formed on the testing electrode terminal 131C;
(4) removing the conductive electroplated layer covered by the shadow mask 12C (23C), wherein the carrier plate 11C formed by the evaporation process is washed away by a washing equipment, such that a block of the conductive electroplated layer 17C covered by the shadow mask 12C is removed by a physical method (such as water rinsing), and the exposed portion includes the testing electrode terminal 131C and the electrode circuit terminal 132C;
(5) setting an enzyme in an enzyme reacting area of the carrier plate (24C), wherein after the enzyme is set on the carrier plate 11C by an enzyme printing equipment 36, the enzyme will be disposed on the carrier plate 11C (or the enzyme reacting area), and the enzyme can be set by a gravure, silk screen, or jet printing or a titration;
(6) attaching a cover film 15C onto the carrier plate 11C (25C), wherein a continuous printing adhesive material (such as a liquid adhesive and an acrylic adhesive) is set on the carrier plate 11C and attached to the cover film 15C correspondingly, and the electrode circuit terminal 132C is exposed to facilitate performing a test;
(7) cutting the plastic film 31 into unit products (26C), wherein the plastic film 31 installed with related components is placed into a cutting machine for performing a cutting procedure to obtain a plurality of complete planar bio-test strip 10C (or blood glucose detector) products.

The present invention adopts the aforementioned manufacturing method, such that the planar bio-test strip can be manufactured by printing (such as a gravure printing or a silk screen printing) a sheet of plastic film of a roll to roll of plastic film, so as to achieve the effects of providing a quick mass production, lowering the cost, simplifying the manufacturing procedure, and improving the quality, stability and market competiveness of the product, and a physical method is applied to obtain the electrically conductivity and achieve the effects of meeting the environmental protection requirements, providing an easy control of impedance and a stable conduction, and providing excellent precision and sensitivity for the physiological measurements.

The present invention improves over the prior art and complies with patent application requirements, and thus is duly filed for the patent application. While the invention has been described by device of specific embodiments, numerous modifications and variations could be made thereto by those generally skilled in the art without departing from the scope and spirit of the invention set forth in the claims.

## Claims

1. A planar bio-test strip 10A, comprising:
a carrier plate 11A, made of a plastic film 31, and printed with a shadow mask 12A, and using an evaporated metal conductor to form a conductive circuit 13A on the shadow mask 12A, and the conductive circuit 13A including a testing electrode terminal 131A and an electrode circuit terminal 132A electrically coupled with each other; and
an enzyme reacting area 16A, coupled at a front end of the testing electrode terminal 131A.

2. A planar bio-test strip 10C, comprising:
a carrier plate 11C, made of a plastic film 31;
a conductive circuit 13C, installed on the carrier plate 11C, and further comprising:
a testing electrode terminal 131C, formed by coating or printing an electrically conductive material; and
an electrode circuit terminal 132C, electrically conducted with the testing electrode terminal 131C, such that after the testing electrode terminal 131C is formed, the electrode circuit terminal 132C has a shadow mask 12C printed onto the carrier plate 11C, and an evaporated metal conductor is formed on the shadow mask 12C; and
an enzyme reacting area 16C, coupled to a front end of the testing electrode terminal 131C.

3. The planar bio-test strip 10C of claim 2, wherein the testing electrode terminal 131C and the evaporated metal conductor form upper and lower coupling electrodes at an end of the bio-test strip 10C away from the enzyme reacting area 16C.

4. The planar bio-test strip of claim 2, wherein the electrically conductive material for forming the testing electrode terminal 131C is a carbon ink or a conductive silver ink.

5. The planar bio-test strip of claim 1 or 2, wherein the carrier plate 11 A, 11C is formed by a single layer of plastic film 31 or attaching a plurality of layers of plastic films 31.

6. The planar bio-test strip of claim 1 or 2, wherein the carrier plate 11A, 11C is made of a PET film, a PC film, a PTFE film, a nylon film, a polycarbonate film, a polystyrene film, a styrene film, an acrylonitrile film or an acrylonitrile butadiene styrene (ABS) film.

7. The planar bio-test strip of claim 1 or 2, wherein the evaporated metal conductive material for forming the conductive circuit 13A, 13C is a metal selected from the collection of gold, silver, copper and aluminum.

8. A method of manufacturing a planar bio-test strip 10A, comprising:
(1) using a roll of cylindrical plastic film 31 as a carrier plate 11 A, and applying a printing method to construct a shadow mask 12A provided for developing a pattern of a testing electrode terminal 131A and a pattern of an electrode circuit terminal 132A;
(2) forming a conductive circuit 13A by an evaporation process, and using the evaporation process to coat a continuous conductive electroplated layer 17A onto the carrier plate 11A that is printed with the shadow mask 12A;
(3) removing the conductive electroplated layer 17A covered by the shadow mask 12A, wherein the conductive electroplated layer 17A covered by the shadow mask 12A is removed by a physical method, and the whole conductive circuit 13A is exposed, and the conductive circuit 13A includes a testing electrode terminal 131A and an electrode circuit terminal 132A electrically conducted with each other; and
(4) setting an enzyme in an enzyme reacting area 16A disposed on the carrier plate 11 A.

9. A method of manufacturing a planar bio-test strip 10C, comprising:
(1) using a roll of cylindrical plastic film 31 as a carrier plate 11C, and printing or coating an electrically conductive material that is not chemically reacted with the enzyme easily on the plastic film 31 to form a testing electrode terminal 131C;
(2) constructing a shadow mask 12C on the carrier plate 11C by a printing method, wherein the shadow mask 12C is coupled to an end of the testing electrode terminal 131C;
(3) covering the electrode circuit terminal 132C and the testing electrode terminal 131C that is disposed away from the enzyme reacting area 16C on the carrier plate 11C printed with the shadow mask 12C by an evaporation process, such that the electrode circuit terminal 132C and the testing electrode terminal 131C are electrically conducted, and coupling electrodes are formed at upper and lower layers of the testing electrode terminal 131C respectively;
(4) removing a block of the conductive electroplated layer 132C covered by the shadow mask 12C on the carrier plate 11C to expose the testing electrode terminal 131C and the electrode circuit terminal 132C, after the evaporation process is completed,; and
(5) setting an enzyme in an enzyme reacting area 16C on the carrier plate 11C.

10. The method of manufacturing a planar bio-test strip 10A, 10C as recited in claim 8 or 9, wherein the shadow mask 12A, 12C is made of a non-toxic water-based oil ink and printed by a gravure printing method or a silk screen printing method.

11. The method of manufacturing a planar bio-test strip 10A, 10C as recited in claim 8 or 9, wherein the evaporated conductive material for forming the conductive, circuit 13A, 13C is a metal selected from the collection of gold, silver, copper and aluminum.

12. The method of manufacturing a planar bio-test strip 10A, 10C as recited in claim 8 or 9, wherein the enzyme is set on the carrier plate 11A, 11C continuously by a gravure printing process, a silk screen printing process, a jet printing process or a titration.

13. The method of manufacturing a planar bio-test strip 10A, 10C as recited in claim 8 or 9, wherein the carrier plate 11A, 11C attached with a cover film 15A, 15C is cut into a plurality of unit products.

14. The method of manufacturing a planar bio-test strip 10C as recited in claim 9, wherein the electrically conductive material for forming the testing electrode terminal 131C is a carbon ink or a conductive silver ink.
